# FASCICULE DE BREVET EUROPEEN

(11) **EP 2 111 387 B1**
(45) Date de publication et mention de la délivrance du brevet: **23.03.2011**
(21) Numéro de dépôt: 08707843.2
(22) Date de dépôt: 09.01.2008
(51) Int. Cl.: C07C 51/09, C07C 59/42, C07C 59/56, A61K 31/231, C07C 67/317, C07C 69/732, C07F 7/18, A61P 17/00

(54) **NOUVEAU PROCEDE DE PREPARATION D'HYDROXY-ACIDES GRAS INSATURES**
NEUES VERFAHREN ZUR HERSTELLUNG UNGESÄTTIGTER HYDROXYFETTSÄUREN
NEW METHOD FOR PREPARING UNSATURATED FATTY HYDROXY-ACIDS

(30) Priorité: 09.01.2007 FR 0700123
(43) Date de publication de la demande: 28.10.2009
(73) Titulaire: Pierre Fabre Dermo-Cosmétique, 92100 Boulogne-Billancourt (FR)
(72) Inventeur: FRISON, Natacha, F-60100 Creil (FR); FOLLEAS, Benoît, F-60300 Senlis (FR); BRAYER, Jean-Louis, F-60440 Nanteuil Le Haudoin (FR)
(74) Mandataire: Ahner, Francis
(86) Numéro de dépôt international: PCT/EP2008/050197
(87) Numéro de publication internationale: WO 2008/084060

(56) Documents cités:
- WO-A-03/022787
- FR-A1- 2 684 988
- LIST B ET AL: "Practical Synthesis of (E)-alpha,beta-Unsaturated Esters from Aldehydes" ADVANCED SYNTHESIS AND CATALYSIS, WILEY-VCH, WEINHEIM, DE, vol. 347, 2005, pages 1558-1560, XP002390820 ISSN: 1615-4150
- VILLIERAS J ET AL: "La Reaction de Wittig-Horner en milieu heterogene VI. Selectivité de la reaction sur des composés bifonctionnels" TETRAHEDRON LETTERS, ELSEVIER, AMSTERDAM, NL, vol. 26, no. 1, 1985, pages 53-56, XP002198720 ISSN: 0040-4039

## Description

La présente invention a pour objet un nouveau procédé de préparation d'hydroxy-acides gras insaturés. Elle a également pour objet de nouveaux hydroxy-acides gras insaturés, ainsi que leur utilisation, notamment dans le domaine cosmétique et pharmaceutique.

De nombreux hydroxy-acides gras saturés sont connus et décrits dans la littérature pour leurs propriétés biologiques et plus particulièrement pour leurs propriétés cosmétiques et pharmacologiques. Par exemple, le principal constituant lipidique de la gelée royale des abeilles est un hydroxy-acide gras insaturé, à savoir l'acide hydroxy-10-décène-2(trans)oïque (Edward E. Smissman et al., 1964, JOC, 29, 3517-3520).

Différents documents de l'état de la technique décrivent des procédés de préparation des hydroxy-acides gras insaturés et de leurs esters (Lee et al., 1993, J. Org. Chem., 58, 2918-2919 ; Hurd et Saunders, 1952, J. Am. Chem. Soc., 74, 5324-5328 ; Krishnamurthy et al., 1989, Indian J.Chem. Sect. A, 28, 288-291 ; Plettner et al., 1995, J. Chem. Ecol., 21, 1017-1030).

Les procédés déjà connus dans l'état de la technique présentent une étape d'oxydation durant laquelle des sels métalliques comme les sels de chrome ou de manganèse sont employés. Or, l'utilisation de sels métalliques présente un certain nombre d'inconvénients. D'une part, au niveau des produits obtenus par lesdits procédés, ces derniers peuvent être contaminés par les sels métalliques et donc leur application cosmétique et/ou pharmacologique est limitée du fait de cette contamination. D'autre part, l'utilisation de sels métalliques entraîne une contamination de l'environnement des industries dans lesquelles la synthèse est effectuée.

D'autres procédés déjà connus dans l'état de la technique montrent qu'une homologation de deux carbones, soit par une réaction de Doebner Knoevenagel, soit par une réaction de Wittig, sur un lactol (lactone partiellement réduite) de petite taille conduit de façon minoritaire à des hydroxy-acides insaturés et de façon majoritaire à des tétrahydropyranyl acétique acide ou tétrahydrofuranyl acétique acide (Ragoussis et al., 1993, Synthesis, 1, 84-86). Ainsi, de tels procédés ne permettent pas d'obtenir des hydroxy-acides gras insaturés dans des quantités satisfaisantes.

La présente invention a pour but de fournir un nouveau procédé de préparation d'hydroxy-acides gras insaturés, dans d'excellentes conditions à la fois en terme de rendement mais aussi de qualité sans trace de contamination, ledit procédé pouvant être transposé au niveau industriel.

La présente invention a pour but de fournir une méthode de synthèse d'hydroxy-acides gras insaturés plus rapide et présentant de meilleurs rendements que les procédés actuellement utilisés, le rendement du procédé de l'invention étant augmenté de 100 à 200% par rapport aux procédés de l'état de la technique.

La présente invention a également pour but de fournir un procédé simplifié par rapport aux procédés de l'état de la technique, et ce notamment en raison d'un nombre réduit d'étapes.

La présente invention a également pour but de fournir un procédé qui ne comprend pas d'étape d'oxydation d'une fonction alcool et qui ne comprend pas l'utilisation de sels de métaux lourds, afin d'éviter les problèmes de contamination avec ces sels de métaux lourds.

La présente invention a également pour but de fournir un procédé écologique comprenant l'utilisation de matières premières renouvelables.

Enfm, la présente invention a pour but de fournir un procédé qui permet d'obtenir toute une gamme de produits, à partir de produits disponibles dans le commerce, dont la plupart sont des matières premières d'origine végétale.

La présente invention concerne un procédé de préparation d'un composé de formule (I) suivante : 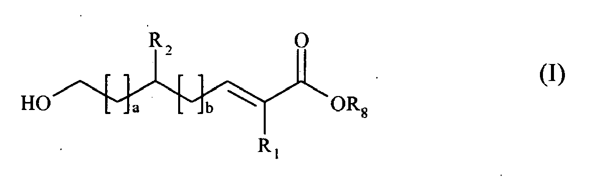 dans laquelle :
- a et b, identiques ou différents, représentent des nombres entiers supérieurs ou égaux à 0, et variant de préférence de 1 à 14 ;
- R₁ représente : H, F, Cl, Br, CF₃, CHF₂, 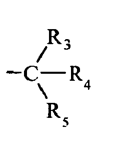 R₃, R₄ et R₅ représentant, indépendamment les uns des autres, soit un atome d'hydrogène soit un groupe alkyle, alkényle ou alkynyle, linéaire ou ramifié, comprenant de 1 à 16 atomes de carbone, éventuellement substitué par un atome d'azote, d'oxygène, de soufre ou un halogène (F, Cl, Br, I),
- R₂ représente soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
- R₈ représente un atome d'hydrogène ou un groupe alkyle R_{b}, linéaire ou ramifié, comprenant de 1 à 6 atomes de carbone, et éventuellement substitué par un ou plusieurs atomes d'oxygène (ester du glycérol),
ledit procédé de préparation comprenant :
- une étape de protection de la fonction alcool du composé de formule (II-a) suivante : 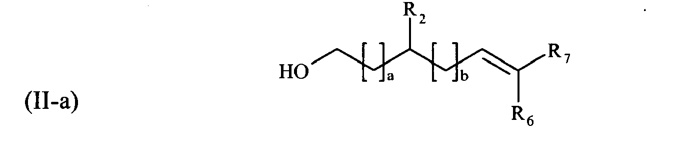
dans laquelle :
- a, b et R₂ sont tels que définis ci-dessus dans la formule (I), et
- R₆ et R₇ représentent, indépendamment les uns des autres, soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
afin d'obtenir un composé de formule (III-a) suivante, dans laquelle la fonction alcool est protégée : 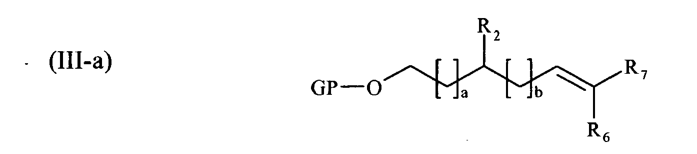 dans laquelle :
- a, b, R₂, R₆ et R₇ sont tels que définis ci-dessus dans la formule (II-a), et
- GP représente un groupe protecteur, pour former avec l'atome d'oxygène adjacent notamment un groupe de type ester ou éther, O-GP étant de préférence un éther silylé, un éther de pyran (THP), un acétate, un propionate ou un pivalate,
   GP correspondant à un groupe protecteur tel que défini dans « Protective Groups in Organic Synthesis » Third edition Theodora GREEN & Peter WUTS Wiley Interscience ISBN 0-471-16019-9 Chapitre 2 pages 17 à 246,
- une étape de coupure oxydante de la double liaison du composé de formule (III-a) telle que définie ci-dessus, pour obtenir un composé de formule (IV-a) suivante : 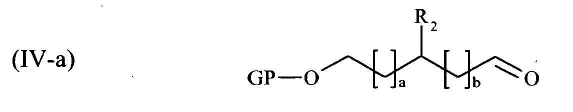 a, b, R₂ et GP étant tels que définis ci-dessus dans la formule (III-a),
   cette étape étant notamment effectuée à l'aide d'oxydants classiques tels que KMnO₄ ou OsO₄ associé au periodate, ou l'ozone, en présence d'un agent réducteur, notamment choisi parmi les dérivés du phosphore ou du soufre,
- et la mise en oeuvre de la réaction de Wittig-Horner par la réaction d'un phosphonate de formule (V) suivante : 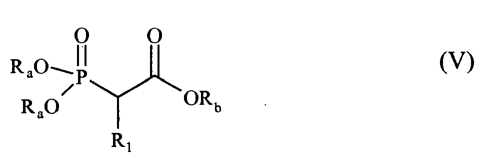
dans laquelle :
- R₁ est tel que défini ci-dessus,
- Rₐ représente un groupe alkyle, linéaire ou ramifié, comprenant de 1 à 6 atomes de carbone, et est de préférence un groupe éthyle ou méthyle, lesdits groupes Rₐ pouvant former un cycle avec les atomes d'oxygène des groupes ORₐ et l'atome de phosphore du groupe P=O, et
- R_{b} représente un groupe alkyle, linéaire ou ramifié, comprenant de 1 à 6 atomes de carbone, et est de préférence un groupe éthyle,
ou la mise en oeuvre, lorsque R₁ = H, de la réaction de Doebner-Knoevenagel par la réaction d'un dérivé d'acide malonique de formule R_{b}OOC-CH₂-COOR_{b}, R_{b} étant tel que défini ci-dessus,
sur le composé de formule (IV-a) tel qu'obtenu à l'issue de l'étape précédente,
ladite étape de réaction de Wittig-Horner ou de Doebner-Knoevenagel étant le cas échéant suivie d'une réaction de saponification puis d'une réaction de déprotection,
afin d'obtenir un composé de formule (I) telle que définie ci-dessus.

Les réactions mises en oeuvre dans le procédé de l'invention permettent de conserver la stéréochimie du produit de départ.

Les composés obtenus et répondant à la formule (I), dans laquelle R₂ est différent de H, sont sous forme de mélanges racémiques ou d'énantiomères.

Les composés de l'invention de formule (I) sont sous la forme de stéréoisomères Z ou E, ou sous la forme d'un mélange de ces différentes formes.

Les matières premières utilisées, c'est-à-dire les composés utilisés répondant à la formule (II-a) susmentionnée, sont des hydroxy-alcènes disponibles commercialement, principalement d'origine végétale, comme le citronellol [CAS : 106-22-9] ou l'acétate de citronellol [CAS : 150-84-5], le 3-butèn-1-ol [CAS : 627-27-0], le trans-3-héxèn-1-ol [CAS : 928-97-2], le 4-pentèn-1-ol [CAS : 821-09-0], le 5-héxèn-1-ol [CAS : 821-41-0], le 9-décèn-1-ol [CAS : 13019-22-2], l'alcool oléique [CAS : 143-28-2], le 10-undécèn-1-ol [CAS : 112-43-6], le *cis*-9-tétradécènol [CAS : 35153-15-2], le *cis*-3-nonèn-1-ol [CAS : 10340-23-5], le *cis*-3-octen-1-ol [CAS : 20125-84-2], le *cis-*4- décèn-1-ol [CAS : 57074-37-0], le cis-5-octèn-1-ol [CAS : 64275-73-6], le *cis*-4-heptèn-1-ol [CAS : 6191-71-5], le *trans*-9-dodécèn-1-ol [CAS : 35237-62-8], le *trans*-5-décèn-1-ol [CAS : 56578-18-8], le *cis*-3-pentèn-1-ol [CAS : 764-38-5], le *cis*-4-héxèn-1-ol [CAS : 928-91-6], le *trans*-4-tridécèn-1-ol [CAS : 75568-02-4], le *cis-*3-heptèn-1-ol [CAS : 1708-81-2], le *cis*-11-tétradécènol [CAS : 34010-15-6], le 2-méthyl-3-butèn-1-ol [CAS : 4516-90-9], le *cis*-7-dodécèn-1-ol [CAS : 20056-92-2], le E,E-8,10-dodécadièn-1-ol [CAS : 33956-49-9], le *cis*-11-hexadécèn-1-ol [CAS : 56683-54-6], le 4-méthyl-3-pentèn-1-ol [CAS : 763-89-« ], le *S*-(-)-β-citronellol [CAS : 7540-51-4], le D-citronellol [CAS : 1117-61-9] et le *cis*-6-nonèn-1-ol [CAS : 35854-86-5].

Toutes ces matières premières commerciales permettent d'accéder à tous les hydroxy-acide gras insaturés depuis 5 atomes de carbones linéaires jusqu'à 18 atomes de carbones linéaires, sans discontinuité.

Selon un mode de réalisation préféré, le procédé de l'invention est caractérisé en ce que l'étape de protection de la fonction alcool est effectuée à une température variant d'environ -40°C à environ 120°C en présence d'un dérivé halogéné, d'un éther d'énol ou d'un anhydride ou d'un chlorure d'acide.

Selon un autre mode de réalisation préféré, le procédé de l'invention est caractérisé en ce que l'étape de coupure oxydante de la double liaison est effectuée à une température variant d'environ -80°C à environ 40°C dans un solvant aprotique.

Dans ce procédé, la première étape consiste en une protection de la fonction alcool primaire et le solvant peut être choisi dans les solvants usuels tels que THP, acétate, pivalate ou SiMe₃.

La seconde étape est une coupure oxydante d'une double liaison. Cette coupure oxydante est effectuée dans un solvant aprotique choisi parmi la liste des solvants usuels tels que le chlorure de méthylène ou le 1,2-dichloroéthane.

L'étape 3 du procédé de l'invention est par exemple une réaction de Wittig-Homer. Cette réaction est une réaction connue de l'homme du métier (Modem Synthetic Reaction, Second edition, Herbet O. House, Wittig Horner reaction pages 682 à 709) et toute condition expérimentale décrite dans l'état de la technique peut être utilisée dans le cadre de la présente invention. A titre d'exemple, la réaction de Wittig-Horner peut être réalisée en présence de triéthylphosphonoacétate (composé de formule (V) dans laquelle Rₐ = R_{b} = Et et R₁ = H) et de carbonate de potassium. Dans ce dernier cas, l'acide est obtenu directement sans hydrolyse supplémentaire.

La dernière étape du procédé de l'invention est une étape de déprotection du groupement protecteur : l'homme du métier sera à même de trouver les conditions expérimentales adéquates à utiliser pour cette étape (« Protective Groups in Organic Synthesis » Third edition Theodora GREEN & Peter WUTS Wiley Interscience ISBN 0-471-16019-9 Chapitre 2 pages 17 à 246). De préférence, cette étape de déprotection est une hydrolyse en milieu légèrement acide.

La présente invention concerne également des composés répondant à l'une des formules suivantes : 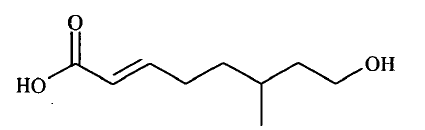 acide 8-hydroxy-6-méthyl-octa-2*t*-énoïque
obtenu à partir du citronellol 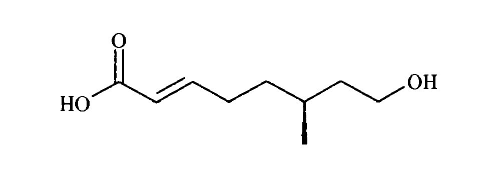 acide (E)-(S)-8-hydroxy-6-méthyl-octa-2-énoïque
obtenu à partir du (S)-(-)-β-citronellol 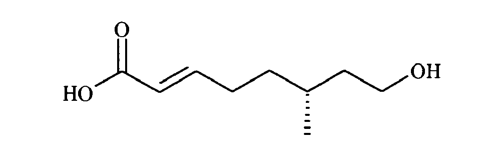 acide (E)-(R)-8-hydroxy-6-méthyl-octa-2-énoïque
obtenu à partir du D-citronellol 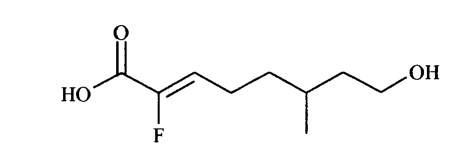 acide 2-fluoro-8-hydroxy-6-méthyl-octa-2-énoïque 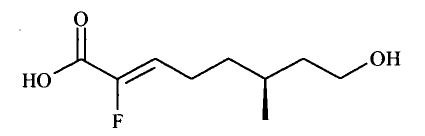 acide (Z)-(S)-2-fluoro-8-hydroxy-6-méthyl-octa-2-énoïque 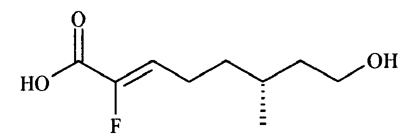 acide (Z)-(R)-2-fluoro-8-hydroxy-6-méthyl-octa-2-énoïque

La présente invention concerne également une composition pharmaceutique, cosmétique ou alimentaire comprenant un composé tel que défini ci-dessus, en association avec un véhicule approprié.

Les composés de l'invention présentent des propriétés biologiques intéressantes, à savoir notamment une activité anti-collagénase.

Le collagène est la protéine la plus abondante et la plus importante du corps humain et de la peau. Cette scléroprotéine représente notamment 75% des protéines du derme auquel elle assure solidité. Le fibroblaste fabrique à partir des acides aminés (hydroxyproline, lysine, proline) des molécules de procollagène qui se transforment en présence de vitamine C en molécules de collagène. Pour former un réseau de fibrilles, le collagène doit créer des liaisons entre ces différentes molécules.

Le renouvellement du collagène change avec l'âge. Le collagène soluble qui donne souplesse et résistance à la peau et aux muqueuses se dégrade de plus en plus rapidement sous l'influence d'une enzyme protéolytique qu'est la collagénase, ce qui entraîne, au niveau du derme, un vieillissement de la structure fibreuse des protéines.

En outre, le collagène insoluble qui entraîne une perte d'élasticité se rigidifie en se polymérisant avec des molécules de glucose grâce à des liaisons multiples difficilement réversibles (phénomène de glycation). Ces liaisons rendent le collagène plus résistant à l'attaque par les collagénases ce qui entraîne une rigidité croissante des fibres de collagène. Ce phénomène de durcissement, caractéristique des tissus cutanés âgés, doit être combattu le plus tôt possible car il augmente la destruction des fibroblastes par les radicaux libres mais aussi la dénaturation des protéines du derme.

Les collagénases sont des enzymes faiblement exprimées dans les conditions physiologiques normales. Leur surexpression lors au vieillissement et en particulier lors de la ménopause chez la femme entraîne une dénaturation plus importante des protéines fibreuses du derme.

Cependant, la destruction des fibres de collagène peut survenir lors d'autres circonstances que le vieillissement. En effet, lors d'une infection bactérienne, les collagénases bactériennes peuvent détruire les fibres de collagène de l'hôte infecté.

De plus, l'invasion tumorale nécessite une dégradation de la membrane basale et de la matrice extra- cellulaire et de toutes les protéines de structure de ces composants parmi lesquelles se trouve le collagène. Ainsi, il a été montré une très nette relation entre le pouvoir invasif des tumeurs et la présence d'activité collagénase dans les tumeurs humaines. On retrouve les collagénases au niveau des cellules tumorales, mais aussi dans les fibroblastes entourant la tumeur. Les cellules épithéliales normales sécrètent un taux très faible de collagénases, alors que ces protéines sont surexprimées par les cellules tumorales invasives ou métastatiques.

D'autres maladies dégénératives présentent une dégénérescence fibrinoïde du collagène et sont également appelées maladies du collagène

L'invention concerne donc l'utilisation des produits susceptibles d'être obtenus par le procédé de l'invention comme agent actif anti-collagénase.

Pour mesurer cette activité anti-collagénase, on peut notamment se référer à la demande de brevet français n° 2 829 491 publiée le 14 mars 2003.

La présente invention concerne également un procédé de préparation d'un composé de formule (I) suivante :
dans laquelle : 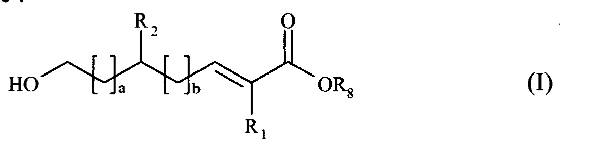
- a et b, identiques ou différents, représentent des nombres entiers supérieurs ou égaux à 0, et variant de préférence de 1 à 14 ;
- R₁ représente : H, F, Cl, Br, CF₃, CHF₂, 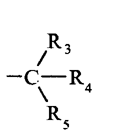 R₃, R₄ et R₅ représentant, indépendamment les uns des autres, soit un atome d'hydrogène soit un groupe alkyle, alkényle ou alkynyle, linéaire ou ramifié, comprenant de 1 à 16 atomes de carbone, éventuellement substitué par un atome d'azote, d'oxygène, de soufre ou un halogène (F, Cl, Br, I),
- R₂ représente soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
- R₈ représente un atome d'hydrogène ou un groupe alkyle R_{b}, linéaire ou ramifié, comprenant de 1 à 6 atomes de carbone, et éventuellement substitué par un ou plusieurs atomes d'oxygène,
ledit procédé de préparation comprenant :
- une étape d'oxydation de la fonction alcool du composé de formule (II-b) suivante : 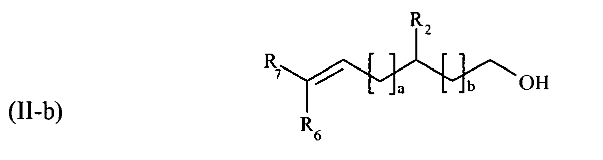 dans laquelle :
   - a, b et R₂ sont tels que définis ci-dessus dans la formule (I), et
   - R₆ et R₇ représentent, indépendamment les uns des autres, soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
      afin d'obtenir un composé de formule (III-b) suivante : 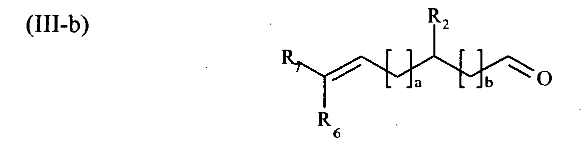 dans laquelle a, b, R₂, R₆ et R₇ sont tels que définis ci-dessus dans la formule (II-b),
   - une étape d'homologation à deux atomes de carbone, et plus particulièrement une étape de mise en oeuvre de la réaction de Wittig-Horner par la réaction d'un phosphonate de formule (V) suivante :
   dans laquelle : 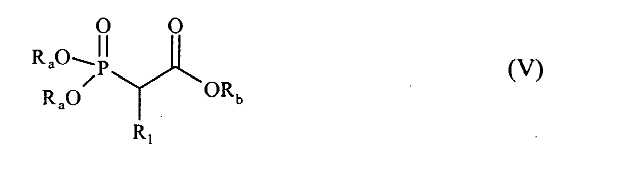
   - R₁ est tel que défini ci-dessus,
   - Rₐ représente un groupe alkyle, linéaire ou ramifié, comprenant de 1 à 6 atomes de carbone, et est de préférence un groupe éthyle ou méthyle, lesdits groupes Rₐ pouvant former un cycle avec les atomes d'oxygène des groupes ORₐ et l'atome de phosphore du groupe P=O, et
   - R_{b} représente un groupe alkyle, linéaire ou ramifié, comprenant de 1 à 6 atomes de carbone, et est de préférence un groupe éthyle,
   ou, lorsque R₁ = H, de la réaction de Doebner-Knoevenagel par la réaction d'un dérivé d'acide malonique de formule R_{b}OOC-CH₂-COOR_{b}, R_{b} étant tel que défini ci-dessus,
   sur le composé de formule (III-b) tel qu'obtenu à l'issue de l'étape précédente,
   ladite étape de réaction de Wittig-Horner ou de Doebner-Knoevenagel étant le cas échéant suivie d'une réaction de saponification puis d'une réaction de déprotection,
   afin d'obtenir un composé de formule (IV-b) suivante : 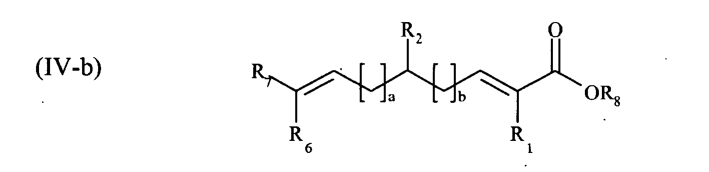 dans laquelle a, b, R₁, R₂, R₆, R₇ et R₈ sont tels que définis ci-dessus,
   - une étape de coupure oxydante de la double liaison du composé de formule (IV-b) telle que définie ci-dessus, suivie d'une étape de réduction,
cette étape étant notamment effectuée à l'aide d'oxydants classiques tels que KMnO₄ ou OsO₄ associé au periodate, ou l'ozone, en présence d'un agent réducteur, notamment choisi parmi les dérivés du bore (hydrures de bore) ou de l'aluminium (hydrures d'aluminium),
afin d'obtenir un composé de formule (I) telle que définie ci-dessus.

Les réactions mises en oeuvre dans le procédé de l'invention permettent de conserver la stéréochimie du produit de départ.

Les composés obtenus et répondant à la formule (I), dans laquelle R₂ est différent de H, sont sous forme de mélanges racémiques ou d'énantiomères.

Les composés de l'invention de formule (I) sont sous la forme de stéréoisomères Z ou E, ou sous la forme d'un mélange de ces différentes formes.

Les matières premières utilisées, c'est-à-dire les composés utilisés répondant à la formule (II-b) susmentionnée, sont des hydroxy-alcènes disponibles commercialement, principalement d'origine végétale (voir liste donnée pour les composés de formule (II-a)).

En ce qui concerne la dernière étape du procédé de l'invention, à savoir l'étape de coupure oxydante, on peut noter qu'il n'était pas évident que le produit obtenu conserve une de ses doubles liaisons.

Selon un mode de réalisation préféré, le procédé de l'invention est caractérisé en ce que l'étape d'oxydation de la fonction alcool est effectuée à l'aide d'un oxydant choisi parmi les oxydes métalliques ou les dérivés de l'iode hypervalent comme le 1-hydroxy-1-oxo-1H-1λ5-benzo[d][1,2]iodoxol-3-one (IBX), le SIBX^{®} (Simaflex) ou le réactif de Dess Martin.

De préférence, dans le procédé de l'invention tel que défini ci-dessus, l'étape d'oxydation est effectuée par la réaction d'Oppenhauer ou la réaction de Swern.

Selon un autre mode de réalisation préféré, le procédé de l'invention est caractérisé en ce que l'étape de coupure oxydante de la double liaison est effectuée à une température variant d'environ -80°C à environ 40°C dans un solvant ou un mélange de solvants contenant au moins une source de protons comme un alcool.

Dans le procédé décrit ci-dessus, la deuxième étape du procédé de l'invention peut être toute méthode d'homologation à deux atomes de carbone comme la réaction de Wittig-Horner ou la réaction de Doebner-Knoevenagel. Ces réactions sont des réactions connues de l'homme du métier (Modem Synthetic Reaction, Second edition, Herbet O. House, Wittig Homer reaction, pages 682 à 709) et Doebner Knoevenagel (646 à 653) et toute condition expérimentale décrite dans l'état de la technique peut être utilisée dans le cadre de la présente invention. A titre d'exemple, la réaction de Wittig-Horner peut être réalisée en présence de triéthylphosphonoacétate et de carbonate de potassium. Dans ce dernier cas, l'acide est obtenu directement sans hydrolyse supplémentaire.

En ce qui concerne l'étape de coupure oxydante, celle-ci est effectuée dans un solvant aprotique choisi parmi la liste des solvants usuels tels que le chlorure de méthylène ou le 1,2-dichloroéthane et le méthanol comme source de protons.

La présente invention concerne également les composés répondant à l'une des formules suivantes : 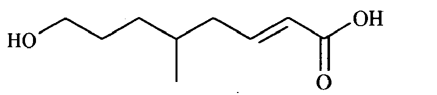 obtenu à partir du citronellol
acide 8-hydroxy-5-méthyl-octa-*2t*-énoïque 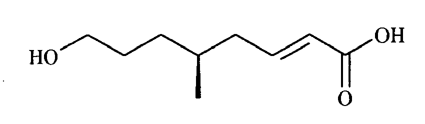 obtenu à partir du (S)-(-)-β-citronellol
acide (E)-(S)-8-hydroxy-5-méthyl-octa-2-énoïque 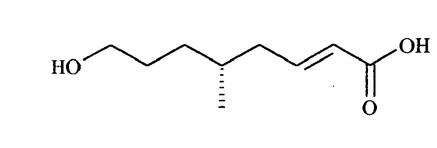 obtenu à partir du D-citronellol
acide (E)-(R)-8-hydroxy-5-méthyl-octa-2-énoïque 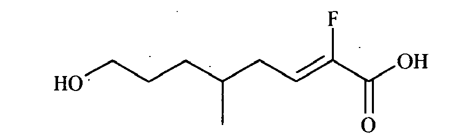 acide 8-hydroxy-5-méthyl-octa-2-fluoro-2-énoïque 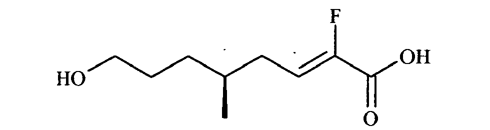 acide (Z)-(S)-2-fluoro-8-hydroxy-5-méthyl-octa-2-énoïque 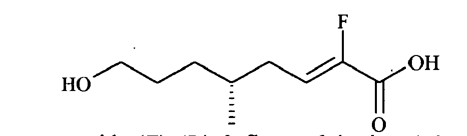 acide (Z)-(R)-2-fluoro-8-hydroxy-5-méthyl-octa-2-énoïque

La présente invention concerne également une composition pharmaceutique, cosmétique ou alimentaire comprenant un composé tel que défini ci-dessus, en association avec un véhicule approprié.

Les composés de l'invention présentent des propriétés biologiques intéressantes, à savoir notamment une activité anti-collagénase telle que décrite ci-dessus.

### PARTIE EXPÉRIMENTALE

### Exemple 1A Mode opératoire pour la synthèse de l'acide 12-hydroxy-dodéca-2t-énoïque : (composé de formule (I) avec R₁ = R₂ = R₈ = H et a+b = 7)

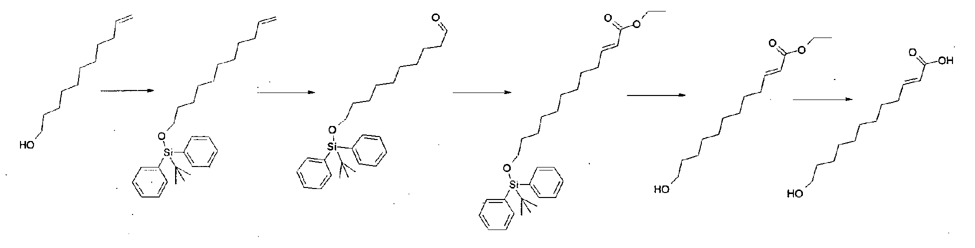

### 1. Etape de protection du 10-undecen-1-ol

Dans un tricol sous flux d'azote, 17g (10 mmol) de 10-undecen-1-ol (Alfa Aesar, référence A14002, Cas [112-43-6]) sont mis en solution dans 30 volumes de dichlorométhane. 17,02g (2,5 eq) d'imidazole sont ajoutés puis 30,2 g (1,1 eq) de tert-butylchlorodiphénylsilane dans 10 volumes de dichlorométhane sont additionnés goutte à goutte. Le milieu réactionnel est agité à température ambiante pendant 6 h. Un suivi CCM permet de contrôler la fin de la réaction. 500 ml d'une solution saturée de NaCl sont ajoutés et le mélange est extrait au dichlorométhane par trois fois. Les phases organiques sont séchées sur MgSO₄, filtrées et concentrées sous vide pour conduire à 44 g d'un liquide. Ce résidu brut est purifié par chromatographie sur colonne de silice, éluée par de l'heptane. 37,5g d'un liquide jaune sont obtenus, soit un rendement de 92%.

Caractérisations :
CCM : Rf= 0,9 (heptane / acétate d'éthyle 95/5)
¹H RMN (300 MHz, CDCl₃) : δ 7,69 (m, 4H) ; 7,42 (m, 6H) ; 5,84 (m, 1H) ; 4,98 (m, 2H) ; 3,68 (t, 2H) ; 2,05 (m, 2H) ; 1,58 (m, 2H) ; 1,28-1,37 (m, 12H) ; 1,09 (s, 9H).

### 2. Etape de coupure oxydante par ozonolyse

Dans un tricol sous flux d'azote, 30 g (73,4 mmol) de 10-undecen-1-ol protégé par le groupement terbutyldiphénylsilyle sont dilués dans 10 volumes de dichlorométhane. Le milieu est refroidi à -78°C. Sous agitation et à -78°C, une pression d'ozone par bullage est imposée pendant 30 minutes (1,1 eq O₃). Le milieu est ensuite purgé sous flux d'azote et 23,1 g (1,2 eq) de triphénylphosphine sont ajoutés. Le milieu est laissé revenir à 0°C, et un contrôle CCM permet de contrôler la fin de la réaction. Le mélange est dilué par du dichlorométhane et hydrolysé par une solution de NaHCO₃ à 5%. La phase organique extraite est lavée à l'eau, puis avec une solution saturée de NaCl. La phase organique est séchée sur MgSO₄, filtrée et concentrée sous vide. Le solide pâteux obtenu est empâté dans un volume d'éther isopropylique pour éliminer l'oxyde de triphénylphosphine. Puis le résidu concentré à sec est purifié sur colonne de silice éluée par un gradient heptane / éther isopropylique. 24,1 g d'un liquide jaune est obtenu, soit un rendement de 80%.

Caractérisations :
CCM : Rf= 0,6 (heptane / acétate d'éthyle 90/10)
¹H RMN (300 MHz, CDCl₃) : δ 9,79 (s, 1H) ; 7,70 (m, 4H) ; 7,42 (m, 6H) ; 3,68 (t, 2H) ; 2,43 (m, 2H) ; 1,60 (m, 2H) ; 1,30 (m, 12H) ; 1,09 (s, 9H).

### 3. Réaction de Wittig-Horner

Dans un tricol sous flux d'azote, 3,5 g (8,5 mmol) d'aldéhyde précédent sont mis en solution dans 10 volumes d'éthanol. 1,77 g (1,5 eq) de carbonate de potassium sont ajoutés et 1,86 ml (1,1 eq) de triéthylphosphonoacétate sont coulés goutte à goutte. Le milieu est ensuite chauffé à 45°C pendant une nuit. Un suivi CCM avec révélation à l'anisaldéhyde permet de contrôler la fin de la réaction. Le milieu est concentré pour éliminer l'éthanol. Puis le résidu est repris à l'eau et à l'acétate d'éthyle. Le mélange est extrait à l'acétate d'éthyle par trois fois puis les phases organiques rassemblées sont lavées par une solution saturée de NaCl, séchées sur MgSO₄, filtrées et concentrées. Le résidu brut est purifié par colonne de silice éluée par un gradient heptane / acétate d'éthyle, pour conduire à 3,1 g d'une huile incolore (soit un rendement de 76%).

Caractérisations :
CCM : Rf= 0,5 (heptane / acétate d'éthyle 90/10)
¹H RMN (300 MHz, CDCl₃) : δ 7,70 (m, 4H) ; 7,36 (m, 6H) ; 6,99 (dt, J= 15,0Hz, 1H) ; 5,83 (dt, J=15,0Hz, 1H) ; 4,21 (q, 2H) ; 3,68 (t, 2H) ; 2,22 (m, 2H) ; 1,59 (m, 2H) ; 1,29-1,47 (m, 15H) ; 1,09 (s, 9H).

### 4. Etape de déprotection de l'alcool

Dans un tricol sous flux d'azote, 830 mg (1,7 mmol) d'hydroxy-acide gras insaturé diprotégé sont mis en solution dans 15 volumes de tétrahydrofurane. Le milieu est refroidi à 0°C puis 2,6 ml (1,5 eq) d'une solution de fluorure de tétrabutylammonium à 1M dans le THF sont coulés goutte à goutte. Le mélange est ensuite agité à température ambiante pendant 1 h. Un suivi CCM permet de contrôler la fin de la réaction. Le milieu réactionnel est alors hydrolysé par une solution saturée de NH₄Cl, puis est extrait à l'acétate d'éthyle par trois fois. Les phases organiques rassemblées sont lavées par une solution saturée de NaCl. Le résidu brut est purifié sur colonne de silice, éluée par un gradient heptane / acétate d'éthyle. 390 mg d'une huile sont obtenus, soit un rendement de 93%.

Caractérisations :
CCM : Rf = 0,3 (heptane / acétate d'éthyle 90/10)
¹H RMN (300 MHz, CDCl₃) δ 6,98 (dt, J= 15,0Hz, 1H) ; 5,82 (dt, J=15,0Hz, 1H) ; 4,20 (q, 2H) ; 3,66 (t, 2H) ; 2,22 (m, 2H) ; 1,58 (m, 2H) ; 1,29-1,49 (m, 15H).
Spectrométrie de masse : [M+Na]⁺ = 265.48 (M calculée 242).

### 5. Etape de saponification

Dans un ballon, 390mg (1.6mmol) d'alcool-ester précédent sont mis en solution dans 10 volumes de tétrahydrofurane puis 1.6ml (2eq) d'une solution de soude 2M sont ajoutés. Le milieu est chauffé à 70°C pendant une nuit. Un suivi CCM permet de contrôler la fin de la réaction. A température ambiante, le pH de la solution est descendu à 2 par ajout d'une solution d'acide chlorhydrique 3M. Le tétrahydrofurane est éliminé sous vide. La phase aqueuse est alors extraite à l'acétate d'éthyle ; les phases organiques sont lavées par une solution saturée de NaCl, séchées sur MgSO₄, filtrées et concentrées sous vide. Le résidu blanc est recristallisé dans l'éther isopropylique pour conduire à 150 mg d'un solide blanc (43% de rendement).

Caractérisations :
CCM : Rf= 0.2 (heptane / acétate d'éthyle 60/40)
¹H RMN (300 MHz, CDCl₃) : 7.09 (dt, J= 15.0Hz, 1H), 5.84 (d, J=15.0Hz, 1H), 3.66 (t, 2H), 2.24 (m, 2H), 1.27-1.61 (m, 14H).
Spectrométrie de masse : [M+Na]⁺ = 237,47 (M calculée 214).

### Exemple 2A Mode opératoire pour la synthèse de l'acide 12-hydroxy-dodéca-2-fluoro-2-énoïque :

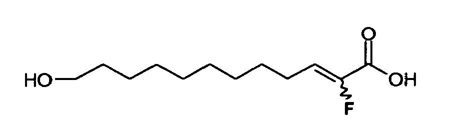

Seule l'étape 3 est modifiée (réaction de Wittig-Horner) : à partir de l'aldéhyde obtenu à l'étape 2 de l'exemple 1, la réaction de Wittig est conduite en présence de triéthylphosphonofluoroacétate (1,1 eq) de carbonate de potassium (1,5 eq) dans l'éthanol à 40°C. Les étapes 4 et 5 (déprotection et saponification) sont conduites comme décrit précédemment. Le solide blanc obtenu après recristallisation est sous forme d'un mélange *cis* / *trans.* L'hydroxy-acide obtenu est un composé de formule (I) avec R₂ = H, R₁ = F et a+b = 7.

Caractérisations :
CCM : Rf = 0,2 (heptane / acétate d'éthyle 60/40)
¹H RMN (300 MHz, CDCl₃) : δ 6.30 (dt, J= 31Hz, 0,08H, forme Z) ; 6,05 (dt, J= 21,0Hz, 0,92H, forme E) ; 3,70 (t, 2H) ; 2,53 (m, 2H) ; 1,32-1,62 (m, 14H).
Spectrométrie de masse : [M+Na]⁺ = 255,53 (M calculée 232).

### Exemple 3A Mode opératoire pour la synthèse de l'acide 8-hydroxy-6-méthyl-octa-2t-énoïque :

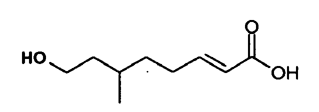

Le protocole de synthèse de l'exemple 1 est appliqué au β-citronellol (Aldrich référence C832001, Cas [106-22-9]). Ceci permet l'obtention de l'acide 8-hydroxy-6-méthyl-octa-2t-énoïque (composé de formule (I) avec R₁ = R₈ = H, R₂ = CH₃, a=1 et b=2).

Caractérisations :
CCM : Rf = 0,1 (heptane / acétate d'éthyle 60/40)
¹H RMN (300 MHz, CDCl₃) : δ 7,09 (dt, J=15 Hz, 1H) ; 5,85 (dt, J= 15Hz, 1H) ; 4,13 (m, 1H) ; 3,72 (m, 2H) ; 2,28 (m, 2H) ; 1,30-1,60 (m, 4H) ; 0,95 (d, 3H).
Spectrométrie de masse : [M+Na]⁺ = 195,46 (M calculée 172).

### Exemple 4A Mode opératoire pour la synthèse de l'acide 8-hydroxy-6-méthyl-octa-2-fluoro-2-énoïque :

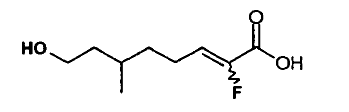

Le protocole de synthèse de l'exemple 2 est appliqué au β-citronellol (Aldrich référence C832001, Cas [106-22-9]). Ceci permet l'obtention de l'acide 8-hydroxy-6-méthyl-octa-2-fluoro-2-énoïque (composé de formule (I) avec R₂ = CH₃, R₁ = F, R₈ = H, a = 1 et b = 2).

Caractérisations :
CCM : Rf = 0,1 (heptane / acétate d'éthyle 60/40)
¹H RMN (300 MHz, CDCl₃) : δ 6,26 (dt, J=33 Hz, 0,5H, forme Z) ; 6,06 (dt, J= 21Hz, 0,5H, forme E) ; 3,73 (m, 2H) ; 2,56 (m, 1H, forme E) ; 2,32 (m, 1H, forme Z) ; 1,36-1,69 (m, 4H) ; 0,95 (d, 3H).
Spectrométrie de masse : [M+Na]⁺ = 213,44 (M calculée 190).

### Exemple 5A Mode opératoire pour la synthèse de l'acide 8-hydroxy-octa-2t-énoïque :

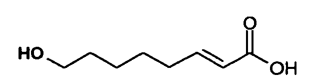

Le protocole de synthèse de l'exemple 1 est appliqué au cis-6-Nonen-1-ol (Aldrich référence W346500, Cas [35854-86-5]). Ceci permet l'obtention de l'acide 8-hydroxy -octa-2*t*-énoïque (composé de formule (I) avec R₁ = R₂ = R₈ = H et a+b = 3).

Caractérisations :
CCM : Rf= 0.1 (heptane / acétate d'éthyle 60/40)
¹H RMN (300 MHz, CDCl₃) : δ 7,09 (dt, J=15,6 Hz, 1H) ; 5,85 (dt, J= 15,6Hz, 1H) ; 3,68 (t, 2H) ; 2,26 (m, 2H) ; 1,40-1,63 (m, 6H).
Spectrométrie de masse : [M+Na]⁺ = 181,10 (M calculée 158).

### Exemple 6A Mode opératoire pour la synthèse de l'acide 8-hydroxy-octa-2-fluoro-2-énoïque :

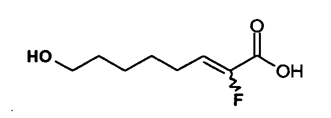

Le protocole de synthèse de l'exemple 2 est appliqué au cis-6-Nonen-1-ol (Aldrich référence W346500, Cas [35854-86-5]). Ceci permet l'obtention de l'acide 8-hydroxy -octa-2-fluoro-2-énoïque (composé de formule (I) avec R₂ = R₈ = H, R₁ = F et a+b = 3).

Caractérisations :
CCM : Rf= 0.1 (heptane / acétate d'éthyle 60/40)
¹H RMN (300 MHz, CDCl₃) : δ 6,26 (dt, J=33 Hz, 1H, forme Z) ; 6,06 (dt, J= 21Hz, 1H, forme E) ; 3,69 (t, 4H) ; 2,58 (m, 2H, forme E) ; 2,30 (m, 2H, forme Z) ; 1,42-1,64 (m, 12H).
Spectrométrie de masse : [M+Na]⁺ = 199,17 (M calculée 176).

### Exemple 1B Mode opératoire pour la synthèse de l'acide 8-hydroxy-5-méthyl-octa-2-énoïque : (composé de formule (I) avec R₂ = CH₃, R₁ = R₈ = H, a = 2 et b = 1)

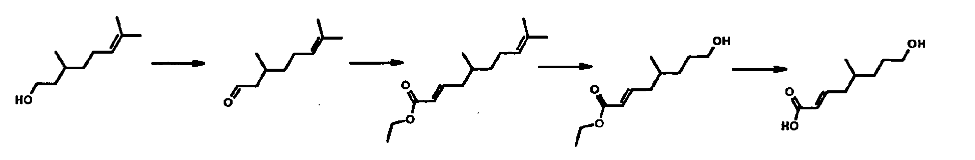

### 1. Etape d'oxydation de l'alcool en aldéhyde

Dans un tricol sous flux d'azote, 20 g (0,128 mol) de β-citronellol (Aldrich référence C832001, Cas [106-22-9]) sont dilués dans 10 volumes de tétrahydrofurane. Lentement, 39,5g (1,1 eq) d'IBX sont ajoutés au milieu réactionnel, qui est alors chauffé une nuit à 50°C. Un contrôle CCM permet de suivre la fin de la réaction. A température ambiante, le milieu est filtré sur célite. Le gâteau est lavé au tétrahydrofurane. Ce filtrat est concentré sous vide. Le résidu obtenu est purifié sur colonne de silice éluée par un mélange heptane / acétate d'éthyle 98/2. 17,6g d'une huile incolore sont obtenus, soit un rendement de 89%.

Caractérisations :
CCM : Rf = 0,7 (heptane / acétate d'éthyle 70/30)
¹H RMN (300 MHz, CDCl₃) : δ 9,77 (s, 1H) ; 5,10 (m, 1H) ; 2,20-2,41 (m, 2H) ; 2,00-2,10 (m, 1H) ; 1,70 (s, 3H) ; 1,62 (s, 3H) ; 1,28-1,40 (m, 4H) ; 1,05 (d, 3H).
Spectrométrie de masse : [M+Na]⁺ = 177,33 (M calculée 154).

### 2. Réaction de Wittig-Horner

Dans un tricol sous flux d'azote, 3 g (19,4 mmol) d'aldéhyde précédent sont mis en solution dans 10 volumes d'éthanol. 2,68 g (1,5 eq) de carbonate de potassium sont ajoutés et 4,79 g (1,1 eq) de triéthylphosphonoacétate sont coulés goutte à goutte. Le milieu est ensuite chauffé à 45°C pendant une nuit. Un suivi CCM avec révélation à l'anisaldéhyde permet de contrôler la fin de la réaction. Le milieu est concentré pour éliminer l'éthanol. Puis le résidu est repris à l'eau et à l'acétate d'éthyle. Le mélange est extrait à l'acétate d'éthyle par trois fois puis les phases organiques rassemblées sont lavées par une solution saturée de NaCl, séchées sur MgSO₄, filtrées et concentrées. Le résidu brut est purifié par colonne de silice éluée par un gradient heptane / acétate d'éthyle, pour conduire à 3,56 g d'une huile incolore (soit un rendement de 82%).

Caractérisations :
CCM : Rf = 0,75 (heptane / acétate d'éthyle 90/10)
¹H RMN (300 MHz, CDCl₃) : δ 6,96 (dt, J=15 Hz, 1H) ; 5,83 (dt, J=15Hz, 1H) ; 5,10 (m, 1H) ; 4,20 (q, 2H) ; 2,21 (m, 1H) ; 1,97-2,08 (m, 4H) ; 1,70 (s, 3H) ; 1,62 (s, 3H) ; 1,31 (t, 3H) ; 1,21-1,30 (m, 2H) ; 0,94 (d, 3H).
Spectrométrie de masse : [M+Na]⁺ = 246,80 (M calculée 224).

### 3. Etape de coupure oxydante

Dans un tricol sous flux d'azote, 3 g (13,3 mmol) du produit précédemment synthétisé sont mis en solution dans un mélange 1/1 (v/v) méthanol / dichlorométhane. Le milieu est refroidi à -78°C. Sous agitation et à -78°C, une pression d'ozone par bullage est imposée pendant 30 minutes. Le milieu est ensuite purgé sous flux d'azote et 4,7 g (9,4 eq) de borohydrure de sodium sont ajoutés par portions, lentement. Le mélange est agité pendant une nuit à température ambiante. Il est ensuite versé sur une solution d'acide chlorhydrique 3M, plongé dans un bain de glace. Le milieu est extrait au dichlorométhane. Les phases organiques sont rassemblées et lavées par une solution saturée de NaHCO₃, puis par une solution saturée de NaCl. Elles sont séchées sur MgSO₄, filtrées et concentrées sous vide.

Caractérisations :
CCM : Rf= 0,2 (heptane / acétate d'éthyle 70/30)
¹H RMN (300 MHz, CDCl₃) : δ 6.94 (dt, *J*=15,6Hz, 1H) ; 5,83 (dt, *J*=15,6Hz, 1H) ; 4.18 (q, 2H) ; 3,65 (t, *J*=6,6Hz, 2H) ; 2.05-2.40 (m, 2H) ; 1,25-1,65 (m, 8H) ; 0,93 (d, 3H).
Spectrométrie de masse : [M+Na]⁺ = 209.46 (M calculée 186).

### 4. Etape de saponification

Le protocole décrit dans l'exemple 1A, 5., est appliqué ici. Ceci permet d'obtenir une huile incolore.

Caractérisations :
CCM : Rf = 0,1 (heptane / acétate d'éthyle 60/40)
¹H RMN (300 MHz, CDCl₃) : δ 7,10 (dt, *J*=15,6Hz*,* 1H) ; 5,84 (dt, *J*=15,6Hz, 1H) ; 3,66 (t, *J*=6,6Hz, 2H) ; 2,05-2,29 (m, 2H) ; 1,22-1,66 (m, 5H) ; 0,92 (d, 3H).
Spectrométrie de masse : [M+Na]⁺ = 195,76 (M calculée 172).

### Exemple 2B Mode opératoire pour la synthèse de l'acide 8-hydroxy-5-méthyl-octa-2-fluoro-2-énoïque :

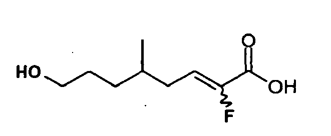

Seule l'étape 2 est modifiée (réaction de Wittig-Horner) : à partir de l'aldéhyde obtenu à l'étape 1 de l'exemple 7, la réaction de Wittig est conduite en présence de triéthylphosphonofluoroacétate (1,1 eq) de carbonate de potassium (1,5 eq) dans l'éthanol à 40°C. Les étapes 3 et 4 (ozonolyse et saponification) sont conduites comme décrit précédemment. L'huile incolore obtenue est sous forme d'un mélange *cis* / *trans.* L'hydroxy-acide obtenu est un composé de formule (I) avec R₈ = H, R₂ = CH₃, R₁ F, a = 2 et b = 1.

Caractérisations :
CCM : Rf = 0,2 (heptane / acétate d'éthyle 60/40)

## Revendications

1. Procédé de préparation d'un composé de formule (I) suivante : 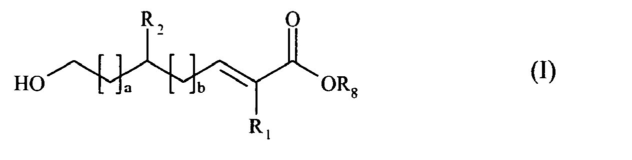 dans laquelle :
• a et b, identiques ou différents, représentent des nombres entiers supérieurs ou égaux à 0, et variant de préférence de 1 à 14 ;
• R₁ représente : H, F, Cl, Br, CF₃, CHF₂, 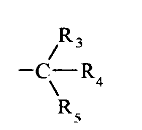 R₃, R₄ et R₅ représentant, indépendamment les uns des autres, soit un atome d'hydrogène soit un groupe alkyle, alkényle ou alkynyle, linéaire ou ramifié, comprenant de 1 à 16 atomes de carbone, éventuellement substitué par un atome d'azote, d'oxygène, de soufre ou un halogène (F, Cl, Br, I),
• R₂ représente soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
• R₈ représente un atome d'hydrogène ou un groupe alkyle R_{b}, linéaire ou ramifié, comprenant de 1 à 6 atomes de carbone, et éventuellement substitué par un ou plusieurs atomes d'oxygène,
ledit procédé de préparation comprenant :
- une étape d'oxydation de la fonction alcool du composé de formule (II-b) suivante : 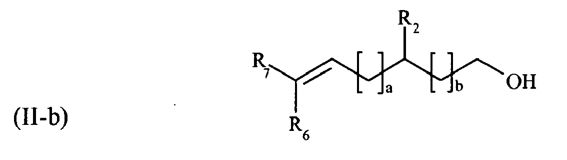
dans laquelle :
- a, b et R₂ sont tels que définis ci-dessus dans la formule (I), et
- R₆ et R₇ représentent, indépendamment les uns des autres, soit un atome d'hydrogène soit un groupe alkyle, alkényle ou alkynyle, linéaire ou ramifié, comprenant de 1 à 16 atomes de carbone, ledit groupe alkyle, alkényle ou alkynyle étant éventuellement substitué par un ou plusieurs atomes d'halogène ou d'oxygène,
afin d'obtenir un composé de formule (III-b) suivante : 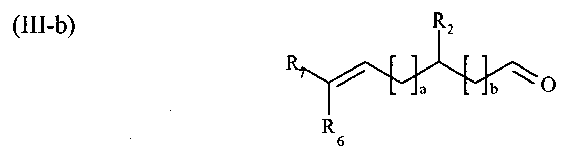 dans laquelle a, b, R₂, R₆ et R₇ sont tels que définis ci-dessus dans la formule (II-b),
- une étape de mise en oeuvre de la réaction de Wittig-Horner par la réaction d'un phosphonate de formule (V) suivante :
dans laquelle : 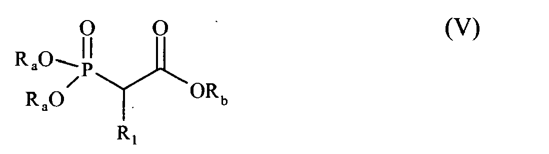
- R₁ est tel que défini ci-dessus,
- Rₐ représente un groupe alkyle, linéaire ou ramifié, comprenant de 1 à 6 atomes de carbone, et est de préférence un groupe éthyle ou méthyle, lesdits groupes Rₐ pouvant former un cycle avec les atomes d'oxygène des groupes ORₐ et l'atome de phosphore du groupe P=O, et
- R_{b} représente un groupe alkyle, linéaire ou ramifié, comprenant de 1 à 6 atomes de carbone, et est de préférence un groupe éthyle,
ou, lorsque R₁ = H, de la réaction de Doebner-Knoevenagel par la réaction d'un dérivé d'acide malonique de formule R_{b}OOC-CH₂-COOR_{b}, R_{b} étant tel que défini ci-dessus,
sur le composé de formule (III-b) tel qu'obtenu à l'issue de l'étape précédente,
ladite étape de réaction de Wittig-Horner ou de Doebner-Knoevenagel étant le cas échéant suivie d'une réaction de saponification puis d'une réaction de déprotection,
afin d'obtenir un composé de formule (IV-b) suivante : 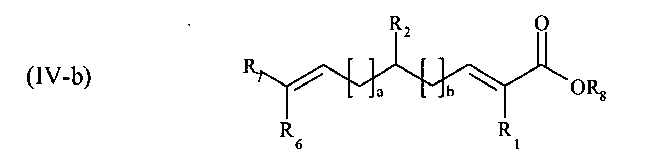 dans laquelle a, b, R₁, R₂, R₆, R₇ et R₈ sont tels que définis ci-dessus,
- une étape de coupure oxydante de la double liaison du composé de formule (IV-b) telle que définie ci-dessus, suivie d'une étape de réduction,
cette étape étant notamment effectuée à l'aide d'oxydants classiques tels que KMnO₄ ou OsO₄ associé au periodate, ou l'ozone, en présence d'un agent réducteur, notamment choisi parmi les dérivés du bore tels que les hydrures de bore ou les dérivés de l'aluminium tels que les hydrures d'aluminium,
afin d'obtenir un composé de formule (I) telle que définie ci-dessus.

2. Procédé selon la revendication 1, **caractérisé en ce que** l'étape d'oxydation de la fonction alcool est effectuée à l'aide d'un oxydant choisi parmi les oxydes métalliques ou les dérivés de l'iode hypervalent comme le 1-hydroxy-1-oxo-1-H-1λ5-benzo[d][1,2]iodoxol-3-one (IBX), le SIBX (Simaflex) ou le réactif de Dess Martin.

3. Procédé selon la revendication 1, **caractérisé en ce que** l'étape d'oxydation est effectuée par la réaction d'Oppenhauer ou la réaction de Swern.

4. Procédé selon l'une quelconque des revendications 1 à 3, **caractérisé en ce que** l'étape de coupure oxydante de la double liaison est effectuée à une température variant d'environ -60°C à environ 40°C dans un solvant aprotique.

5. Composés répondant à l'une des formules suivantes :

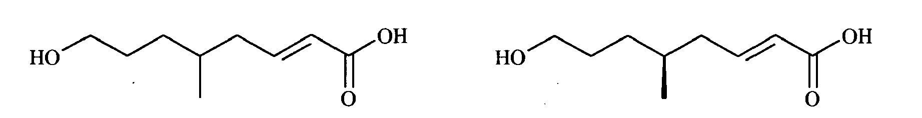

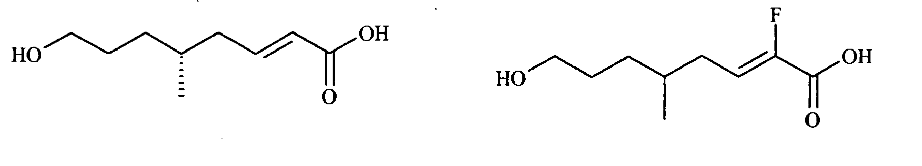

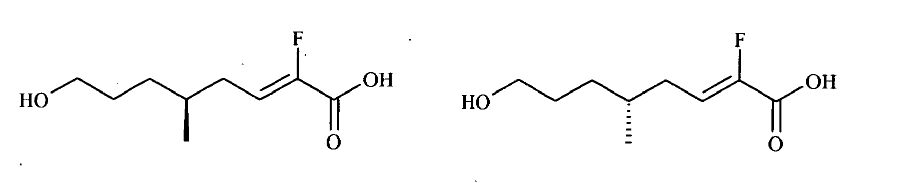

6. Composition pharmaceutique, cosmétique ou alimentaire comprenant un composé selon la revendication 5, en association avec un véhicule approprié.

## Claims

1. Method for preparing a compound having formula (I) hereinafter: 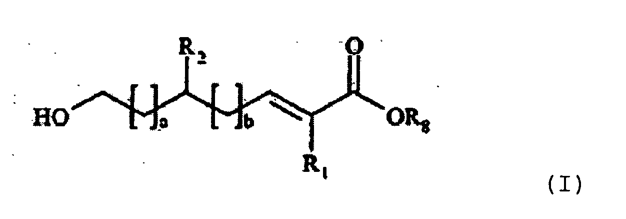 wherein:
-- a and b, identical or different, represent integers greater than or equal to 0, and preferably varying from 1 to 14;
-- R₁ represents:
H, F, Cl, Br, CF₃, CHF₂, 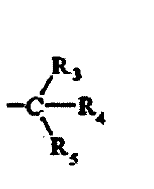
R₃, R₄ and R₅ representing, independently from each other, either a hydrogen atom or a linear or branched alkyl, alkenyl or alkynyl group, comprising 1 to 16 carbon atoms, optionally substituted by a nitrogen, oxygen, sulphur atom or a halogen (F, Cl, Br, I),
-- R₂ represents either a hydrogen atom or a linear or branched alkyl, alkenyl or alkynyl group, comprising 1 to 16 carbon atoms, said alkyl, alkenyl or alkynyl group being optionally substituted by one or a plurality of halogen or oxygen atoms,
-- R₈ represents a hydrogen atom or a linear or branched alkyl group R_{b}, comprising 1 to 6 carbon atoms, optionally substituted by one or a plurality of oxygen atoms,
said preparation method comprising:
- a step for oxidising the alcohol functional group of the compound having formula (II-b) hereinafter: 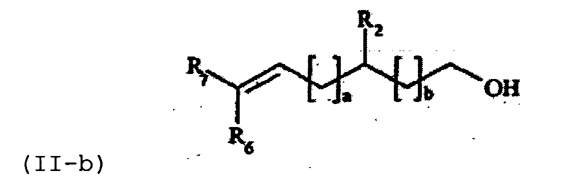
wherein:
- a, b and R₂ are as defined above in formula (I), and
- R₆ and R₇ represent, independently from each other, either a hydrogen atom or a linear or branched alkyl, alkenyl or alkynyl group, comprising 1 to 16 carbon atoms, said alkyl, alkenyl or alkynyl group being optionally substituted by one or a plurality of halogen or oxygen atoms,
to obtain a compound having formula (III-b) hereinafter: 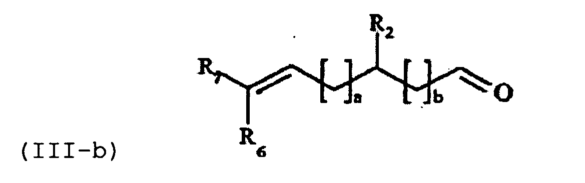 wherein a, b, R₂, R₆ and R₇ are as defined above in formula (II-b),
- a step for carrying out the Wittig-Horner reaction of a phosphonate having formula (V) hereinafter: 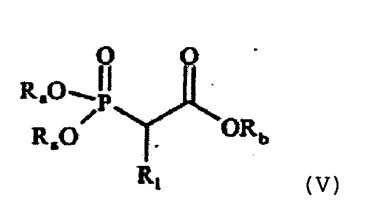
wherein:
- R₁ is as defined above,
- Rₐ represents a linear or branched alkyl group, comprising 1 to 6 carbon atoms, and is preferably an ethyl or methyl group, said Rₐ groups being capable of forming a ring with the oxygen atoms from the ORₐ groups and the phosphorus atom from the P=O group, and
- R_{b} represents a linear or branched alkyl group, comprising 1 to 6 carbon atoms, and is preferably an ethyl group,
or, if R₁ = H, the Doebner-Knoevenagel reaction by reacting a malonic acid derivative having the formula R_{b}OOC-CH₂-COOR_{b}, where R_{b} is as defined above,
on the compound having formula (III-b) obtained from the previous step,
said step of Wittig-Horner or Doebner-Knoevenagel reaction being optionally followed by a saponification reaction and then a deprotection reaction,
to obtain a compound having formula (IV-b) hereinafter: 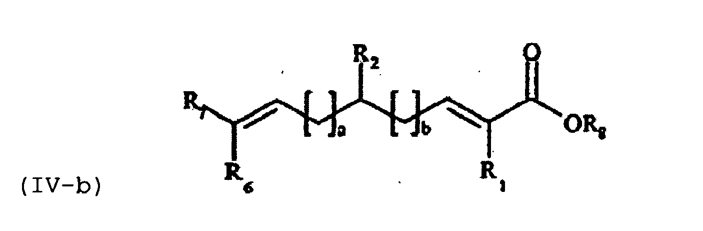 wherein a, b, R₁, R₂, R₆, R₇ and R₈ are as defined above,
- a step for the oxidative splitting of the double bond of the compound having formula (IV-b) as defined above, followed by a reduction step,
said step being particularly performed using conventional oxidants such as KMnO₄ or OsO₄ associated with periodate, or ozone, in the presence of a reducing agent, particularly selected from boron derivatives such as boron hydrides or aluminium derivatives such as aluminium hydrides,
to obtain a compound having formula (I) as defined above.

2. Method according to claim 1, **characterised in that** the step for oxidising the alcohol functional group is performed using an oxidant selected from metal oxides or hypervalent iodine derivatives such as 1-hydroxy-1-oxo-1H-1λ5-benzo[d][1,2]iodoxol-3-one (IBX), SIBX (Simaflex) or Dess Martin reagent.

3. Method according to claim 1, **characterised in that** the oxidation step is performed by means of an Oppenhauer reaction or Swern reaction.

4. Method according to any of claims 1 to 3, **characterised in that** the step for the oxidative splitting of the double bond is performed at a temperature varying from approximately -60°C to approximately 40°C in an aprotic solvent.

5. Compounds according to any of the following formulas:

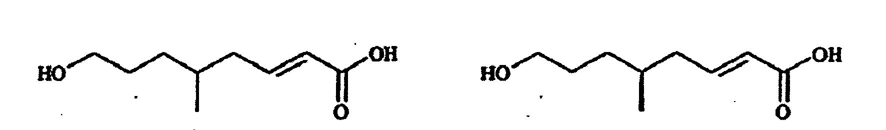

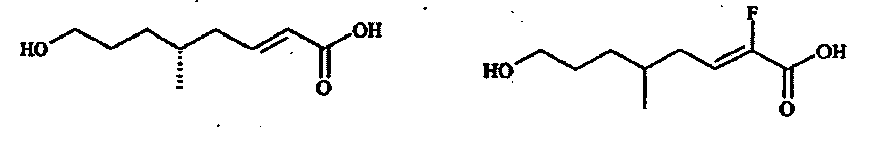

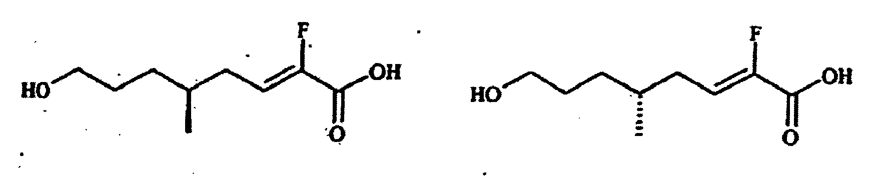

6. Pharmaceutical, cosmetic or nutritional composition comprising a compound according to claim 5, in association with a suitable vehicle.

## Patentansprüche

1. Verfahren zur Herstellung einer Verbindung der folgenden Formel (I) 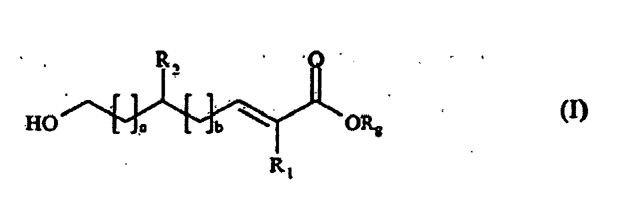 in der:
• a und b, identisch oder verschieden, eine ganze Zahl größer oder gleich 0 darstellen und vorzugsweise von 1 bis 14 variieren;
• R₁ darstellt: H, F, Cl, Br, CF₃, CHF₂, 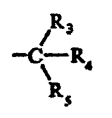 R₃, R₄ und R₅ unabhängig voneinander entweder ein Wasserstoffatom oder eine lineare oder verzweigte Alkyl-, Alkenyl- oder Alkinylgruppe darstellen, die 1 bis 16 Kohlenstoffatome umfasst und gegebenenfalls durch ein Stickstoff-, Sauerstoff-, Schwefelatom oder ein Halogen (F, Cl, Br, I) substituiert ist;
• R₂ entweder ein Wasserstoffatom oder eine lineare oder verzweigte Alkyl-, Alkenyl- oder Alkinylgruppe darstellt, die 1 bis 16 Kohlenstoffatome umfasst, wobei die Alkyl-, Alkenyl- oder Alkinylgruppe gegebenenfalls durch ein oder mehrere Halogen- oder Sauerstoffatome substituiert ist,
• R₈ ein Wasserstoffatom oder eine lineare oder verzweigte Alkylgruppe R_{b} darstellt, die 1 bis 6 Kohlenstoffatome umfasst und gegebenenfalls durch ein oder mehrere Sauerstoffatome substituiert ist,
wobei das Herstellungsverfahren umfasst:
- einen Schritt der Oxidation der Alkoholfunktion der Verbindung der folgenden Formel (II-b) 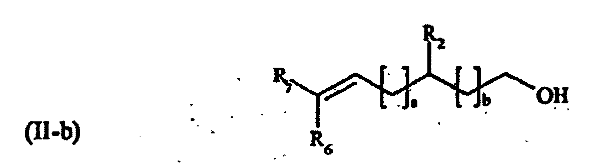
in der:
- a, b und R₂ wie vorstehend in der Formel (I) definiert sind und
- R₆ und R₇ unabhängig voneinander entweder ein Wasserstoffatom oder eine lineare oder verzweigte Alkyl-, Alkenyl- oder Alkinylgruppe darstellen, die 1 bis 16 Kohlenstoffatome umfasst, wobei die Alkyl-, Alkenyl- oder Alkinylgruppe gegebenenfalls durch ein oder mehrere Halogen- oder Sauerstoffatome substituiert ist, um eine Verbindung der folgenden Formel (III-b) zu erhalten: 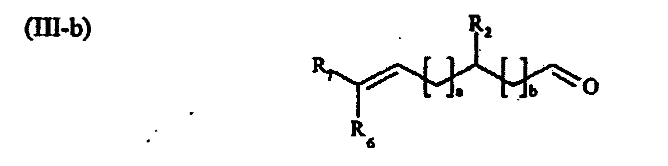 in der a, b, R₂, R₆ und R₇ wie vorstehend in der Formel (II-b) definiert sind,
- einen Schritt der Durchführung der Wittig-Horner-Reaktion durch Umsetzung eines Phosphonats der folgenden Formel (V): 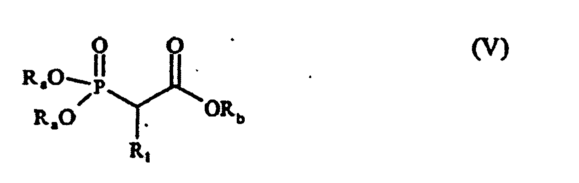
in der:
- R₁ wie vorstehend definiert ist,
- Rₐ eine lineare oder verzweigte Alkylgruppe darstellt, die 1 bis 6 Kohlenstoffatome umfasst und vorzugsweise eine Ethyl- oder Methylgruppe ist, wobei die Gruppen Rₐ einen Cyclus mit den Sauerstoffatomen der Gruppen ORₐ und dem Phosphoratom der Gruppe P=O bilden können, und
- R_{b} eine lineare oder verzweigte Alkylgruppe darstellt, die 1 bis 6 Kohlenstoffatome umfasst und vorzugsweise eine Ethylgruppe ist, oder, wenn R₁ = H, der Doebner-Knoevenagel-Reaktion durch Umsetzung eines Derivates von Malonsäure der Formel R_{b}OOC-CH₂-COOR_{b}, wobei R_{b} wie vorstehend definiert ist,
mit der Verbindung der Formel (III-b), wie am Ende des vorstehenden Schritts erhalten,
wobei dem Schritt der Wittig-Horner oder der Doebner-Knoevenagel-Reaktion gegebenenfalls eine Verseifungsreaktion, dann eine Entschützungsreaktion folgt,
um eine Verbindung der folgenden Formel (IV-b) zu erhalten: 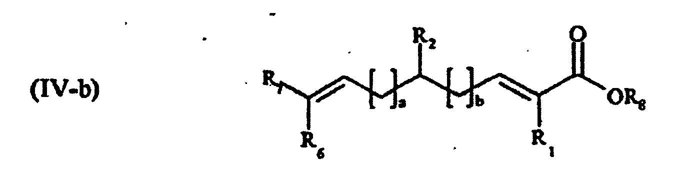 in der a, b, R₁, R₂, R₆, R₇ und R₈ wie vorstehend definiert sind,
- einen Schritt der oxidativen Spaltung der Doppelbindung der Verbindung der Formel (IV-b), wie vorstehend definiert, gefolgt von einem Reduktionsschritt,
wobei dieser Schritt insbesondere mit Hilfe von klassischen Oxidationsmitteln wie KMnO₄ oder OsO₄ in Verbindung mit Periodat oder Ozon in Gegenwart eines Reduktionsmittels durchgeführt wird, das insbesondere aus Bor-Derivaten, wie Borhydriden, oder Aluminium-Derivaten, wie Aluminiumhydriden, ausgewählt ist,
um eine Verbindung der Formel (I), wie vorstehend definiert, zu erhalten.

2. Verfahren nach Anspruch 1, **dadurch gekennzeichnet, dass** der Schritt der Oxidation der Alkohol-Funktion mit Hilfe eines Oxidationsmittels bewirkt wird, das aus Metalloxiden oder Derivaten von hypervalentem Iod, wie 1-Hydroxy-1-oxo-1H-1λ5-benzo[d][1,2]iodoxol-3-on (IBX), SIBX (Simaflex) oder dem Dess-Martin-Reagens ausgewählt ist.

3. Verfahren nach Anspruch 1, **dadurch gekennzeichnet, dass** der Oxidationsschritt durch die Oppenhauer-Reaktion oder die Swern-Reaktion durchgeführt wird.

4. Verfahren nach irgendeinem der Ansprüche 1 bis 3, **dadurch gekennzeichnet dass** der Schritt der oxidativen Spaltung der Doppelbindung in einem aprotischen Lösungsmittel bei einer Temperatur bewirkt wird, die von etwa -60 °C bis etwa 40 °C variiert.

5. Verbindungen, die einer der folgenden Formeln entsprechen:

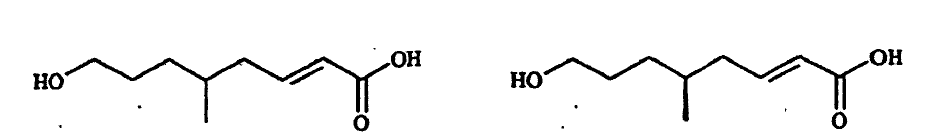

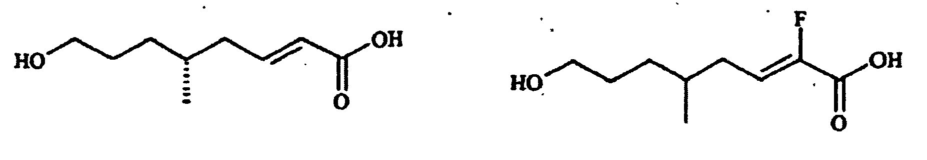

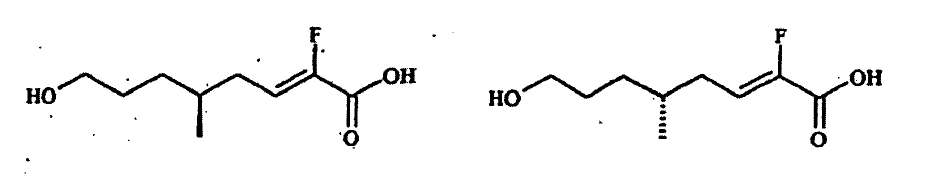

6. Pharmazeutische, kosmetische oder NahrungsmittelZusammensetzung, die eine Verbindung nach Anspruch 5 in Verbindung mit einem geeigneten Vehikel umfasst.
